**Europäisches Patentamt**

**European Patent Office**

**Office européen des brevets**

(19)

(11) Veröffentlichungsnummer : **0 419 954 B1**

(12)

# EUROPÄISCHE PATENTSCHRIFT

(45) Veröffentlichungstag der Patentschrift :
**02.12.92 Patentblatt 92/49**

(51) Int. Cl.⁵ : **C07C 303/32,** C07C 309/10,
E21B 43/22

(21) Anmeldenummer : **90117618.0**

(22) Anmeldetag : **13.09.90**

(54) **Verfahren zur Herstellung kältestabiler alkanolisch-wässriger Lösungen von Polyethersulfonaten.**

(30) Priorität : **23.09.89 DE 3931840**

(43) Veröffentlichungstag der Anmeldung :
**03.04.91 Patentblatt 91/14**

(45) Bekanntmachung des Hinweises auf die
Patenterteilung :
**02.12.92 Patentblatt 92/49**

(84) Benannte Vertragsstaaten :
**DE FR GB SE**

(56) Entgegenhaltungen :
**EP-A- 0 116 929**

(56) Entgegenhaltungen :
**EP-A- 0 156 601**
**EP-A- 0 206 678**
**EP-A- 0 251 250**
**FR-A- 2 353 526**

(73) Patentinhaber : **BASF Aktiengesellschaft**
**Carl-Bosch-Strasse 38**
**W-6700 Ludwigshafen (DE)**

(72) Erfinder : **Greif, Norbert, Dr.**
**Im Woogtal 3**
**W-6719 Bobenheim (DE)**
Erfinder : **Oppenlaender, Knut, Dr.**
**Otto-Dill-Strasse 23**
**W-6700 Ludwigshafen (DE)**

## Beschreibung

Die vorliegende Erfindung betrifft ein verbessertes Verfahren zur Herstellung kältestabiler alkanolisch-wäßriger Lösungen von Polyethersulfonaten der allgemeinen Formel I

$$R-(O-A)_n-SO_3M \qquad I$$

in der

R für eine Alkyl- oder Alkenylgruppe mit 8 bis 25 C-Atomen oder eine Alkylphenylgruppe mit 8 bis 18 C-Atomen im Alkylrest steht,

A eine 1,2-Alkylengruppe mit 2 bis 4 C-Atomen bezeichnet,

n einen Wert zwischen 1 und 15 hat und

M ein Alkalimetall- oder ein Ammoniumkation, welches durch $C_1$-$C_4$-Alkylgruppen oder $C_1$-$C_4$-Hydroxyalkylgruppen substituiert sein kann, bedeutet, durch Umsetzung von Polyetherhalogeniden der allgemeinen Formel II

$$R-(O-A)_n-X \qquad II$$

in der X für Chlor oder Brom steht, mit einer wäßrigen Lösung eines Alkalimetall- oder Ammoniumsulfits und Verdünnen der Reaktionslösung mit einem $C_4$-$C_8$-Alkanol.

Weiterhin betrifft die Erfindung die Verwendung der so hergestellten Lösungen von Polyethersulfonaten I als Tensid-Lösungen bei der tertiären Erdölförderung.

Konzentrierte wäßrige Lösungen von Polyethersulfonaten, die zur Viskositätserniedrigung mit kurzkettigen Alkoholen verdünnt sind, sind bekannt, beispielsweise aus der EP-A 156 601 und der EP-A 206 678.

Diese alkoholisch-wäßrigen Tensid-Lösungen haben, wenn es sich um technische Lösungen handelt, oftmals den Nachteil, bei Temperaturabsenkung schon auf 20 bis 15°C wachsartig fest zu werden, da die Polyethersulfonate auskristallisieren. Solche Mischungen sind praktisch nicht mehr förderbar und können Leitungen verstopfen und Lagertanks blockieren. Zudem gehen die Polyethersulfonate auch nur wieder schwer in Lösung.

Dieser Nachteil ist besonders schwerwiegend für die Verwendung dieser Tensid-Lösungen bei der Erdölförderung in kälteren Klimazonen der Erde und in kalten Meeren wie beispielsweise der Nordsee.

Aufgabe der vorliegenden Erfindung war es somit, kältestabile Polyethersulfonat-Lösungen bereitzustellen.

Demgemäß wurde das eingangs definierte Verfahren gefunden, welches dadurch gekennzeichnet ist, daß man das Reaktionsgemisch im Anschluß an die Umsetzung auf 60 bis 150°C bei 1 bis 5 bar erwärmt und die sich hierbei ausbildende überwiegend wäßrige Phase abtrennt.

Beim Erwärmen des Reaktionsgemisches auf 60 bis 150°C bei 1 bis 5 bar, vorzugsweise 90 bis 105°C bei Normaldruck, bilden sich eine überwiegend wäßrige und eine überwiegend organische Phase aus. Die überwiegend wäßrige Phase enthält neben Wasser einen Teil des Alkanols und den Großteil der anorganischen Salze, vornehmlich Halogenide wie Natriumchlorid, die aus der Umsetzung von II mit der Sulfit-Lösung stammen und für das kälteinstabile Verhalten der Lösungen von I verantwortlich sind. Die überwiegend organische Phase enthält neben Alkanol und etwas Wasser den Rest der anorganischen Salze und die nahezu vollständige Menge des Polyethersulfonates I.

Die Phasentrennung wird zweckmäßigerweise auch in der Wärme im angegebenen Temperaturbereich vorgenommen. Nach Abtrennen der überwiegend wäßrigen Phase kann die überwiegend organische Phase mit Wasser auf einen Tensid-Gehalt von üblicherweise 25 bis 35 Gew.-% eingestellt und der weiteren Verwendung zugeführt werden.

Der Rest R der Polyethersulfonate I steht insbesondere für eine Alkylgruppe mit 8 bis 25 C-Atomen, vorzugsweise 10 bis 22 C-Atomen, beispielsweise n-Octyl, 2-Ethylhexyl, n-Nonyl, iso-Nonyl, n-Decyl, n-Undecyl, n-Dedecyl, n-Tridecyl, iso-Tridecyl, Myristyl, Cetyl, Stearyl und Eicosyl, daneben aber auch für eine Alkenylgruppe mit 8 bis 25 C-Atomen, vorzugsweise 10 bis 22 C-Atomen, beispielsweise Oleyl, Linolyl und Linolenyl, oder für eine Alkylphenylgruppe mit 8 bis 18 C-Atomen, vorzugsweise 10 bis 16 C-Atomen im Alkylrest, beispielsweise p-Decylphenyl, p-Dodecylphenyl, p-Myristylphenyl, p-Cetylphenyl und p-Stearylphenyl.

Die Gruppierung A bezeichnet vorzugsweise eine Ethylengruppe, daneben aber auch eine Propylen-, 1,2-Butylen- oder 2,3-Butylengruppe.

Der Alkoxylierungsgrad n hat einen Wert zwischen 1 und 15, vorzugsweise zwischen 2 und 6.

Das Kation M bedeutet ein Alkalimetallkation, insbesondere Natrium oder Kalium, oder ein Ammoniumkation, welches vorzugsweise unsubstituiert ist, daneben aber auch durch $C_1$-$C_4$-Alkylgruppen oder $C_1$-$C_4$-Hydroxyalkylgruppen substituiert sein kann, beispielsweise Trimethylammonium, Triethylammonium, Triethanolammonium oder Triisopropanolammonium.

Die Polyetherhalogenide II werden in der Regel in bekannter Weise aus den entsprechenden Polyetheralkoholen durch Reaktion mit einem Halogenierungsmittel, vorzugsweise einem Chlorierungsmittel wie Thionylchlorid oder Sulfurylchlorid, hergestellt. Die Polyetheralkohole ihrerseits sind durch Alkoxylierung von beispielsweise Fettalkoholen oder Oxoalkoholen leicht zugänglich.

Als Alkalimetall- oder Ammoniumsulfite eignen sich hauptsächlich Sulfite, Hydrogensulfite oder Disulfite, als Kationen kommen die für M bezeichneten in Betracht. Als Beispiele sind Natriumsulfit, Kaliumsulfit, Ammoniumsulfit, Natriumhydrogensulfit, Kaliumhydrogensulfit und Ammoniumhydrogensulfit zu nennen.

Die Umsetzung der Polyetherhalogenide II zu den Sulfonaten I mit der wäßrigen Lösung des Sulfits wird in an sich bekannter Weise vorgenommen. Man arbeitet in der Regel bei Temperaturen von 130 bis 200°C und bei einem Druck von 2 bis 5 bar. Man erhält so üblicherweise hochviskose wäßrige Tensid-Lösungen mit einem hohen Gehalt an anorganischen Salzen. Der Gehalt an I wird normalerweise auf 25 bis 40 Gew.-% eingestellt.

Als $C_4$-$C_8$-Alkanole eignen sich beispielsweise n-Butanol, iso-Butanol, n-Pentanol, iso-Pentanol, n-Hexanol, n-Heptanol und n-Octanol. Besonders gute Ergebnisse erzielt man mit einem Pentanol oder einem Gemisch verschiedener Pentanol-Isomerer, vorzugsweise n-Pentanol und iso-Pentanol oder mit Mischungen hieraus. Technisches Pentanol stellt solche eine Mischung mit einer Zusammensetzung von ca. 70 Gew.-% des n-Isomeren und ca. 30 Gew.-% des iso-Isomeren dar.

Man setzt das Alkanol der Reaktionslösung während oder unmittelbar im Anschluß an die Umsetzung von II mit Sulfit zu. Man kann aber auch so arbeiten, daß man das Alkanol der schon auf 60 bis 150°C erwärmten wäßrigen Lösung von I zugibt und mit ihr erst in der Wärme vermischt. Hierbei wählt man die Menge an Alkanol so, daß sie in der Regel 50 bis 250 Gew.-%, bezogen auf die Menge des Tensids I, beträgt; die besten Ergebnisse erzielt man mit 100 bis 150 Gew.-% Alkanol.

Die nach dem erfindungsgemäßen Verfahren hergestellten Lösungen der Polyethersulfonate I eignen sich in hervorragender Weise als Tensid-Lösungen bei der tertiären Erdölförderung, bei der man durch das Einspritzen von Lösungen oberflächenaktiver Stoffe in die unterirdischen Erdöllagerstätten die Grenzflächenspannung zwischen Öl und umgebendem Wasser herabsetzt und somit die Ausbeute an gefördertem Erdöl erhöhen kann.

Das erfindungsgemäße Verfahren liefert dünnflüssige konzentrierte Polyethersulfonat-Lösungen, die bis zu Temperaturen von 5 - 0°C kältestabil sind. Es stellt eine einfache und effektive Reinigungsmethode für technische Lösungen der Polyethersulfonate I dar, mit der man ihren Gehalt an anorganischen Salzen senken kann.

Beispiel 1

Eine 27 gew.-%ige wäßrigen Lösung von Natrium-p-dodecylphenyloxy-di(ethylenoxy)-ethansulfonat wurde durch Umsetzung von p-Dodecylphenyloxy-di(ethylenoxy)-ethylchlorid mit wäßriger Natriumsulfit-Lösung bei 160°C und 3 bar hergestellt. Die Lösung enthielt 6,4 Gew.-% Natriumchlorid.

Zu 500 g dieser Lösung wurden bei 95 bis 98°C und bei Normaldruck unter intensivem Rühren 194 g technisches Pentanol gegeben. Das Gemisch bildete anschließend schnell zwei Phasen, die sich problemlos trennen ließen. Die organische Phase (556 g) enthielt 2,4 Gew.-% NaCl, die wäßrige Phase (138 g) 13,3 Gew.-% NaCl. Die organische Phase wurde mit Wasser auf einen Tensidgehalt von 30 Gew.-% verdünnt. Diese Lösung war bei 5 bis 0°C kälte- und lagerstabil.

Beispiel 2

Eine 35 gew.-%ige wäßrige Lösung von Natriumtridecyloxy-tri(ethylenoxy)-ethansulfonat wurde durch Umsetzung von Tridecyloxy-tri(ethylenoxy)-ethylchlorid mit wäßriger Natriumsulfit-Lösung bei 160°C und 3 bar hergestellt. Die Lösung enthielt 5,1 Gew.-% Natriumchlorid.

Zu 2000 g dieser Lösung wurden bei 100°C und bei Normaldruck unter intensivem Rühren 700 g technisches Pentanol gegeben. Das Gemisch bildete anschließend schnell zwei Phasen, die sich problemlos trennen ließen. Die organische Phase (2137 g) enthielt 1,0 Gew.-% NaCl, die wäßrige Phase (557 g) 14,3 Gew.-% NaCl. Die organische Phase wurde mit Wasser auf einen Tensidgehalt von 30 Gew.-% verdünnt. Diese Lösung war bei 5°C kälte- und lagerstabil.

**Patentansprüche**

1. Verfahren zur Herstellung kältestabiler alkanolisch-wäßriger Lösungen von Polyethersulfonaten der allgemeinen Formel I

$$R-(O-A)_n-SO_3M \qquad I$$

in der

R für eine Alkyl- oder Alkenylgruppe mit 8 bis 25 C-Atomen oder eine Alkylphenylgruppe mit 8 bis 18 C-Atomen im Alkylrest steht,

A eine 1,2-Alkylengruppe mit 2 bis 4 C-Atomen bezeichnet,

n einen Wert zwischen 1 und 15 hat und

M ein Alkalimetall- oder ein Ammoniumkation, welches durch $C_1$-$C_4$-Alkylgruppen oder $C_1$-$C_4$-Hydroxyalkylgruppen substituiert sein kann, bedeutet,

durch Umsetzung von Polyetherhalogeniden der allgemeinen Formel II

$$R-(O-A)_n-X \qquad II$$

in der X für Chlor oder Brom steht, mit einer wäßrigen Lösung eines Alkalimetall- oder Ammoniumsulfits und Verdünnen der Reaktionslösung mit einem $C_4$-$C_8$-Alkanol, dadurch gekennzeichnet, daß man das Reaktionsgemisch im Anschluß an die Umsetzung auf 60 bis 150°C bei 1 bis 5 bar erwärmt so daß eine Phasentrennung erfolgt, und die sich hierbei ausbildende überwiegend wäßrige Phase abtrennt.

2. Verfahren zur Herstellung kältestabiler alkanolisch-wäßriger Lösungen von Polyethersulfonaten I nach Anspruch 1, dadurch gekennzeichnet, daß man als $C_4$-$C_8$-Alkanol ein Pentanol oder ein Gemisch verschiedener Pentanol-Isomerer verwendet.

3. Verfahren nach Anspruch 1 oder 2, dadurch gekennzeichnet, daß man es auf die Herstellung kältestabiler alkanolisch-wäßriger Lösungen von Polyethersulfonaten I anwendet, bei denen

R für eine Alkylgruppe mit 10 bis 22 C-Atomen steht,

A eine Ethylengruppe bezeichnet,

n einen Wert zwischen 2 und 6 hat und

M ein Natrium-, Kalium- oder unsubstituiertes Ammoniumkation bedeutet.

4. Verwendung der gemäß den Ansprüchen 1 bis 3 hergestellten kältestabilen alkanolisch-wäßrigen Lösungen von Polyethersulfonaten I als Tensid-Lösungen bei der tertiären Erdölförderung.

## Claims

1. A process for preparing alkanolic aqueous solutions, which are stable at low temperature, of polyethersulfonates of the formula I

$$R-(O-A)_n-SO_3M \qquad I$$

where

R is alkyl or alkenyl of 8 to 25 carbon atoms or alkylphenyl with 8 to 18 carbon atoms in the alkyl,

A is 1,2-alkylene of 2 to 4 carbon atoms,

n is from 1 to 15, and

M is an alkali metal or ammonium which can be substituted by $C_1$-$C_4$-alkyl or $C_1$-$C_4$-hydroxyalkyl groups,

by reacting polyether halides of the formula II

$$R-(O-A)_n-X \qquad II$$

where x is chlorine or bromine, with an aqueous solution of an alkali metal or ammonum sulfite and diluting the reaction solution with a $C_4$-$C_8$-alkanol, wherein the mixture is, when the reaction is complete, heated to from 60 to 150°C under from 1 to 5 bar so that phase separation occurs, and the predominantly aqueous phase produced by this is separated off.

2. A process for preparing alkanolic aqueous solutions, which are stable at low temperature, of polyethersulfonates I as claimed in claim 1, wherein a pentanol or a mixture of various pentanol isomers is used as $C_4$-$C_8$-alkanol.

3. A process as claimed in claim 1 or 2, which is used for the preparation of alkanolic aqueous solutions, which are stable at low temperature, of polyethersulfonates I where

R is alkyl of 10 to 22 carbon atoms,

A is ethylene,

n is from 2 to 6, and

M is sodium, potassium or unsubstituted ammonium.

4. The use of alkanolic aqueous solutions, which are stable at low temperature, of polyether-sulfonates I prepared as claimed in claims 1 to 3, as surfactant solutions in tertiary crude oil production.

## Revendications

1. Procédé de préparation de solutions hydroalcanoliques stables au froid de polyéthersulfonates de formule générale I

$$R-(O-A)_n-SO_3M \qquad I$$

dans laquelle

R est mis pour un groupement alkyle ou alcényle à 8-25 atomes de carbone ou pour un groupement alkylphényle à 8-18 atomes de carbone dans le reste alkyle,

A représente un groupement 1,2-alkylène à 2-4 atomes de carbone,

n a une valeur comprise entre 1 et 15 et

M représente un cation de métal alcalin ou d'ammonium, qui peut être substitué par des groupements alkyle en $C_1$-$C_4$ ou par des groupements hydroxyalkyle en $C_1$-$C_4$,

par réaction d'halogénures de polyéther de formule générale II

$$R-(O-A)_n-X \qquad II$$

dans laquelle X est mis pour un atome de chlore ou de brome, avec une solution aqueuse d'un sulfite de métal alcalin ou d'ammonium et dilution de la solution réactionnelle avec un alcanol en $C_4$-$C_8$, caractérisé en ce qu'à la suite de la réaction, on chauffe le mélange réactionnel à une température de 60 à 150°C sous 1 à 5 bar, de sorte qu'il se produise une séparation de phases, et on sépare la phase essentiellement aqueuse qui se forme dans ces conditions.

2. Procédé de préparation de solutions hydroalcanoliques stables au froid de polyéthersulfonates I selon la revendication 1, caractérisé en ce qu'on utilise, comme alcanol en $C_4$-$C_8$, un pentanol ou un mélange de différents isomères du pentanol.

3. Procédé selon la revendication 1 ou 2, caractérisé en ce qu'on l'applique à la préparation de solutions hydro-alcanoliques stables au froid de polyéthersulfonates I dans lesquels

R est mis pour un groupement alkyle à 10-22 atomes de carbone,

A représente un groupement éthylène,

n a une valeur comprise entre 2 et 6 et

M représente un cation de sodium, de potassium ou d'ammonium non substitué.

4. Utilisation des solutions hydro-alcanoliques stables au froid de polyéthersulfonates I, préparées selon l'une quelconque des revendications 1 à 3, comme solutions de tensio-actives dans l'extraction tertiaire de pétrole.